Europäisches Patentamt

European Patent Office

Office européen des brevets

(11) Veröffentlichungsnummer: **0 256 163**
**A1**

(19)

(12) **EUROPÄISCHE PATENTANMELDUNG**

(21) Anmeldenummer: 86111552.5

(22) Anmeldetag: **20.08.86**

(51) Int. Cl.⁴: **G01N 27/66**

(43) Veröffentlichungstag der Anmeldung:
**24.02.88 Patentblatt 88/08**

(84) Benannte Vertragsstaaten:
**AT CH DE FR GB IT LI NL SE**

(71) Anmelder: **Drägerwerk Aktiengesellschaft**
**Moislinger Allee 53-55**
**D-2400 Lübeck 1(DE)**

Anmelder: **Akademie der Wissenschaften der**
**DDR**
**Otto-Nuschke-Strasse 22/23**
**DDR-1086 Berlin(DD)**

(72) Erfinder: **Adler, Joachim, Dipl.-Chem.**
**Dortmunder Strasse 62**
**DDR-7025 Leipzig(DD)**
Erfinder: **Döring, Hans Rüdiger, Dipl.-Phys.**
**Ludwigstrasse 131**
**DDR-7050 Leipzig(DD)**
Erfinder: **Grosse, Hans-Jörg, Dr. sc. nat.**
**Dipl.-Phys.**
**Comeniusstrasse 34**
**DDR-7050 Leipzig(DE)**

(74) Vertreter: **Patentanwälte Beetz sen. - Beetz**
**jun. Timpe - Siegfried - Schmitt-Fumian**
**Steinsdorfstrasse 10**
**D-8000 München 22(DE)**

(54) Verfahren zum Nachweis phosphorhaltiger Substanzen in Luft.

(57) Ein Verfahren zum selektiven, kontinuierlichen und hochempfindlichen Nachweis phosphorhaltiger anorganischer und organischer Verbindungen in Luft an Arbeitsplätzen und in der freien Atmosphäre soll derart verbessert werden, daß spezielle phosphorhaltige Substanzen ohne zusätzliche Reagenzien und mit einer Nachweisgrenze von weniger als 5 ppb bestimmt werden können.

Dazu wird auf die phosphorhaltigen Verbindungen in Gegenwart von Sauerstoff und/ oder Wasserdampf thermische, Strahlungs-oder chemische Energie übertragen.

Fig.1

## Verfahren zum Nachweis phosphorhaltiger Substanzen in Luft

### Anwendungsgebiet der Erfindung

Die Erfindung betrifft ein Verfahren zum selektiven, kontinuierlichen und hochempfindlichen Nachweis phosphorhaltiger anorganischer und organischer Verbindungen in Luft an Arbeitsplätzen (z.B. bei der Herstellung und Anwendung von Pflanzenschutzmitteln, Einsatz von Dotiergasen in der Mikroelektronik) und in der freien Atmosphäre (Kontrolle lufthygienischer Grenzwerte).

### Charakteristik der bekannten technischen Lösungen

Es sind zahlreiche chemische, physikochemische, biochemische und physikalische Verfahren zum Nachweis von Phosphorverbindungen in Gasen und Dämpfen bekannt. Unter dem Aspekt einer kontinuierlichen Analysenführung sind dabei vor allem die verschiedenen Varianten nach der Schönemann-Reaktion, das enzymatische Verfahren, der Nachweis mittels elektrochemischer Zellen und die Flammenphotometrie von Interesse. In letzter Zeit finden Verfahren auf der Grundlage der dispersiven UV-und IR-Spektroskopie zunehmend Interesse.

Bei der Schönemann-Reaktion werden nach einer naßchemischen Oxidation der phosphorhaltigen Verbindung aus bestimmten aromatischen Aminen farbige Produkte gebildet. Da diese Reaktion eine Gruppenreaktion ist, bestehen Querempfindlichkeiten gegen Aldehyde, Säurehalogenide, Säureanhydride und Arylsulfonyl halogenide. Die Reaktion verläuft im Minutenbereich, somit ist nur eine quasikontinuierliche analysenführung möglich. Die Nachweisgrenze beträgt etwa 1 bis 2 μg phosphorhaltiger Verbindung pro Milliliter Lösung. Eine Erhöhung der Empfindlichkeit wird erzielt, wenn statt der aromatischen Amine Indol eingesetzt wird, bei dessen Oxidation Indoxyl als Zwischenprodukt entsteht, dessen bei UV-Bestrahlung auftretende grünblaue Fluoreszenz photometrisch ausgewertet wird. Auf diese Weise kann die Nachweisgrenze auf 0,05 bis 0,1 μg phosphorhaltige Verbindung pro Milliliter Lösung gesenkt werden. Eine weitere Modifikation der Schönemann-Reaktion ist die Auswertung der grünblauen Chemolumineszenz, die bei der Oxidation von Luminol auftritt. Die Nachweisgrenze ist jedoch höhe als bei der fluoreszenzvariante.

Die biochemischen Verfahren beruhen darauf, daß die phosphorhaltigen Verbindungen, d.h. Phosphororganika, inhibierend auf bestimmte Enzyme (z.B. Cholinesterase) einwirken, die geeignete Substrate (z.B. Cholin) spalten können. Die pro Zeiteinheit gespaltene Substratmenge wird kolorimetrisch, fluorometrisch oder elektrochemisch gemessen. Die große Selektivität, die sich aus dem ablaufenden Metabolismus (Wahl von Enzym und Substrat) ergibt, kann jedoch durch YStörreaktionen bei der Messung der gespaltenen Substratmenge stark reduziert werden (z.B. Querempfindlichkeit gegen alle sauer reagierenden Gase bei kalorimetrischen Messungen). Die hohe Empfindlichkeit leitet sich aus der Tatsache ab, daß eine bestimmte Enzymmenge eine Substratmenge spalten kann, die bis zu 10 Millionen Mal größer ist. Dazu ist jedoch eine Inkubationszeit nötig, die im allgemeinen im Minutenbereich liegt, so daß sich die Forderungen einer hohen Empfindlichkeit und einer schnellen Meßwertbildung einander ausschließen.

Zu den physikochemischen Verfahren zählt die elektrochemische Zelle: das Ruhepotential einer elektrochemichen Zelle erhöht sich beim Einwirken von z.B. Phosphororganika (bezeichnet als innere oder spontane Elektrolyse), die durch eine PTFE-Membran in das Element permeieren. Duch die Wahl des Elektrolyten kann der Detektor der nachzuweisenden phosphorhaltigen Verbindung angepaßt werden. Weiter von Vorteil sind die kleinen Abmessungen der Zelle, kein Reagenzverbrauch und keine Vorrichtungen für den Meßgastransport (z.B. Pumpen). Das Eindringen der nachzuweisenden Phsophorverbindungen in die Zelle durch Permeation hat jedoch auch Nachteile: starke Temperatur-und Molekülstrukturabhängigkeiten der Permeationsraten und eine vergleichweise hohe Ansprechzeit des Detektors. Die Nachweisgrenze von Geräten, die mit solchen elektrochemischen Zellen ausgerüstet sind, betragen zur Zeit 50 bis 80 ppb Phosphoroganika in Luft, d.h., sie liegen um 1 bis 2 Größenordnungen über den zu erfassenden subletalen Konzentrationen.

Ein weiteres physikochemisches Verfahren ist die Flammenphotometrie. In einem aus der Gaschromatographie bekannten Flammenionisationsdetektor wird die Phosphorverbindung verbrannt und die dabei entstehende Phosphoremissionsbande bei 526 nm photometriert. Dieser Detektor zeichnet sich durch geringe Größe und einfachen Aufbau aus, notwendig ist jedoch eine Brenngasflasche (im allgemeinen Wasserstoff). Für kontinuierlich betriebene Geräte beträgt die Nachweisgrenze für Phosphorverbindungen im allgemeinen etwa 0,5 bis 0,1 ppm.

Zu den physikalischen Verfahren zählt die dispersive UV-und IR-Spektroskopie. Optische Geräte, die auf diesen Verfahren aufbebaut sind (Infrarot-bzw. Raman-Spektroskopie unter Verwendung von Lasern in den Wellenlängenbereichen von 8 bis 13 $\mu$m bzw. 300 bis 400 $\mu$m), sind zwar komplizierter im Aufbau und kostspieliger im Betrieb, haben aber den Vorteil, daß das Messen von Konzentrationen phosphorhaltiger Verbindungen in größeren Entfernungen vom Gerät möglich ist. Dadurch kann u.a. der Nachteil der im allgmeinen ungünstigeren nachweigrenzen (ca. 20 ppb bis 1 ppm) zum Teil kompensiert werden.

Schließlich wird noch vorgeschlagen (DD-WP 75 827), toxische Komponenten in Luft und anderen Gasgemischen in einem besonderen Reaktionsraum bei einer solchen Temperatur mit einem als feste Phase vorliegenden Reagens umzusetzen, daß dampfförmige Reaktionsprodukte entstehen, die bei der Abkühlung Aerosole bilden, deren Konzentration in einer Ionisationskammer gemessen wird.

## Ziel der Erfindung

Ziel der Erfindung ist der schnelle, spezifische und kontinuierliche Nachweis von phosphorhaltigen anorganischen und organischen Verbindungen in Luft.

## Darlegung des Wesens der Erfindung

Der Erfindung liegt die Aufgabe zugrunde, das Verfahren zum aerosolionisationsgasanalytischen Nachweis toxischer Komponenten in Luft so zu verändern, daß spezielle phosphorhaltige Substanzen ohne zusätzliche Reagenzien und mit einer Nachweisgrenze von weniger als 5 ppb bestimmt werden können.

Das erfindungemäße Verfahren besteht in der Umwandlung der phosphorhaltigen Verbindungen in Aerosole und der ionisationsgasanalytischen Bestimmung der Aerolsolkonzentration und ist dadurch gekennzeichnet, daß auf die phosphorhaltigen Verbindungen in Gegenwart von Sauerstoff und/oder Wasserdampf thermische, Stahlungs-oder chemische Energie übertragen wird. Bei entsprechender Eichung ist der Meßwert ein Maß für die Konzentration der phosphorhaltigen Verbindugen in Luft.

Wir die für die Umwandlung der phosphorhaltigen Verbindung in ein Aerosol erforderliche Energie aus thermischen oder Strahlungsquellen zugeführt, wird kein zusätzliches Reagens benötigt. Bei Einsatz thermischer Energie ist in der Kondensationszone ein thermischer Gradient von vornehmlich -(200 - 300) $K.cm^{-1}.s^{-1}$ einzuhalten. Wird chemische Energie verwendet, kann das hierzu benötigte Reagens (z.B. Ozon) aus dem im Meßgas enthaltenen Sauerstoff erzeugt werden, indem ein Teil des Meßgases abgetrennt, von phosphorhaltigen Verbindungen gereinigt und durch eine stabile Wechselspannungsentladung geleitet oder mit UV $\perp$ C-Strahlung behandelt wird. Dadurch entfällt auch in diesem Falle eine Bevorratung des Reagens.

Durch Variation der zugeführten thermischen Energie sind Rückschlüsse auf die Bindungsstärken des Phosphors in den phosphorhaltigen Verbindungen und somit auch auf deren Struktur möglich, so daß innerhalb der Gruppe der Phosphorverbindungen bestimmte Komponenten ausgeblendet werden können.

Da die Aerosolteilchen nur durch chemische Kondensation der Reaktionsprodukte des Phosphors entstehen, bewirken solche Elemente, wie Schwefel, Kohlenstoff, Sauerstoff, Wasserstoff und Halogene, die neben Phosphor noch in der nachzuweisenden Luft vorhanden sein können, keinen oder nur einen vernachlässigbar geringen Einfluß auf das Meßsignal. Natürlich vorhandene Aerosole in der Luft (Stäube, Nebel) können durch geeignete Aerosolfilter aus der Meßprobe entfernt werden. Sollte das nicht möglich sein, weil die nachzuweisenden phosphorhaltigen Verbindungen ebenfalls in Aerosolform vorliegen, dann müssen erforderlichenfalls verschiedene Messungen kombiniert werden.

Die Bestimmung der Konzentration der erzeugten Aerosole erfolgt vorteilhafterweise mit einer Ionisationskammer mit eingebauter Strahlungsquelle, die sich bei entsprechender Dimensionierung (z.B. gemäß DD-WP 140 688) durch eine sehr hohe Empfindlichkeit gegen Aerosole auszeichnet, so daß eine günstige Nachweisgrenze des Verfahrens errreicht wird.

Die Vorteile der Erfindung bestehen darin, daß das Verfahren kontinuierlich durchführbar ist, eine sehr günstige Nachweisgrenze bei hoher Selektivität besitzt und keine Reagensbevorratung erfordert. Das Verfahren läßt sich mit einfachen und robusten, d.h. wenig störanfälligen Mitteln realisieren.

## Ausführungsbeispiel

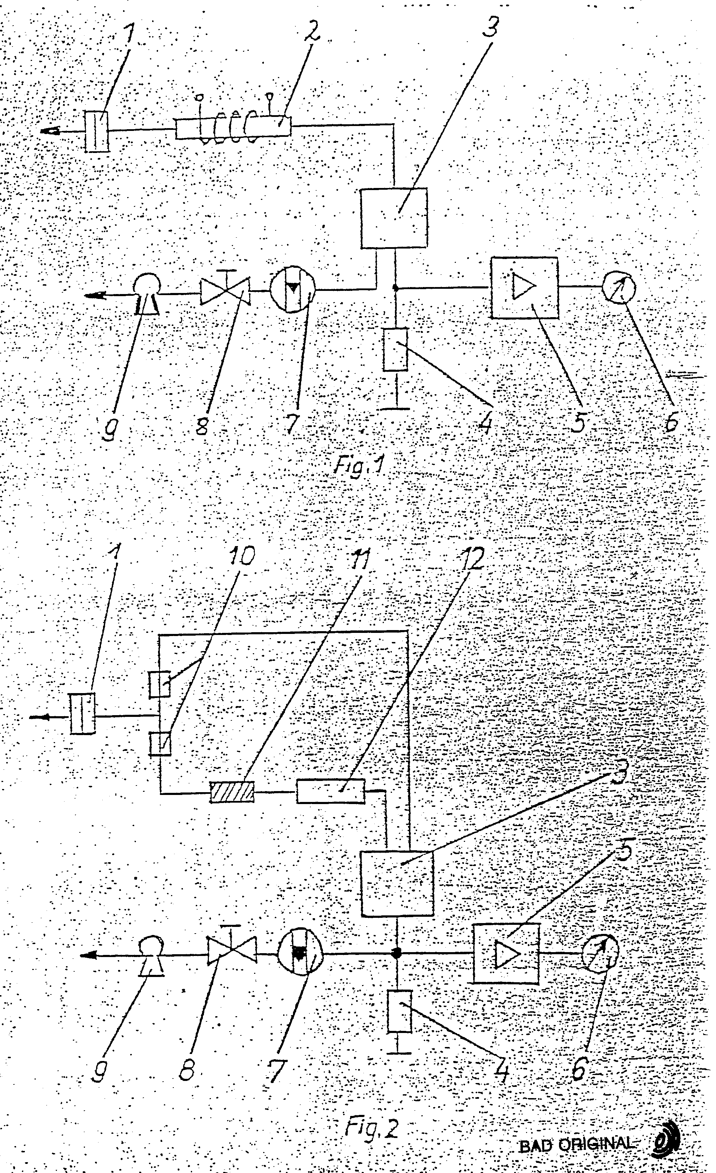

Figur 1 zeigt das Verfahrensschema bei Verwendung thermischer Energie und Figur 2 bei Verwendung chemische Energie.

1. Die Meßprobe (Luft mit der nachzuweisenden phosphorhaltigen Verbindung) gelangt durch ein Aerosolfilter 1, in dem die im Meßgas vorhandenen Aerosole (Stäube, Nebel) zurückgehalten werden, in einen Ofen 2. Der Ofen 2 besteht aus einem elektrisch beheizten Quarzrohr mit entsprechender thermischer Isolierung. Hier erfolgt bei Temperaturen oberhalb von ca. 800 °C mit Hilfe des in der Luft vorhandenen Sauerstoffs die Umwandlung der phosphorhaltigen Verbindung. In der sich dem Ofen 2 anschließenden Abkühlzone kondensieren die Reaktionsprodukte zu Aerosolen, die in einer Ionisationskammer 3 mit eingebauter Strahlenquelle nachgewiesen werden. Die Änderung des Stromes in der Ionisationskammer 3 bei Anwesenheit der Aerosole bewirkt eine Änderung des Spannungsabfalls am Arbeitswiderstand 4, die über einen Impedanzwandler 5 durch ein Instrument oder einen Schreiber 6 angezeigt wird. Der durch eine Pumpe 9 hervorgerufene Luftdurchsatz wird mittels Ventil 8 eingestellt und am Strömungsmesser 7 angezeigt.

Bei einem Luftdurchsatz von 50 l.h$^{-1}$, einer Ofentemperatur von 850 °C und einer Ionisationskammer mit Kr-85-Strahlenquelle (Kammerspannung + 50 V) können bei kontinuierlichem Betrieb folgende Ergebnisse erzielt werden:

Nachweisgrenze: < 1 ppb $PH_3$ oder Methylphosphonsäuredimethylester

Totzeit:< 1 s

95 %ige Ansprechzeit:<10 s

95 %ige Rückstellzeit:<5 Minuten

Querempfindlichkeiten: gegen $SO_2$ keine, gegen Kohlenwasserstoffe $10^3$ ... $10^5$.

2. Der Meßprobe (wiederum Luft mit der nachzuweisnden phosporhaltigen Verbindung) wird nach Passieren des Aerosolfilters 1 mittels Kapillaren 10 ein Teilstrom (ca. 10 % des Meßgasstroms) entnommen, der im Filter 11 (Absorptionsfilter) von allen phosphorhaltigen Verbindungen gereinigt und dann in ein Bestrahlungsgefäß 12 aus Quarz geleitet wird. Unter der Einwirkung der UV $\perp$ C-Strahlung einer Quecksilberdampfniederdrucklampe wird aus Luftsauerstoff Ozon gebildet. Beide Gasströme werden anschließend in der Ionisationskammer 2 mit eingebauter Strahlungsquelle vermischt, so daß die chemische Reaktion des Ozons mit den phosphorhaltigen Verbindungen zur Bildung von Aerosolen führt. Die sich einstellende Ionisationsstromverminderung wird nach der Spannungsabfall methode am Widerstand 4 mit Hilfe des Impedanzwandlers 5 am Instrument 6 angezeigt.

Mit einer nach dem beschriebenen Schema aufgebauten kontinuierlich betriebenen Apparatur können bei den Parametern:

-Luftdurchsatz: 45 1.h$^{-1}$

-Ozonkonzentration: ca. 25 ppm

-zylindrische Ionisationskammer mit äußerem Elektrodenradius von 3 cm und Kr-85-Strahlungsquelle (Kammerspannung + 40 V)

folgende Ergebnisse erzielt werden:

-Nachweisgrenze: ca. 0,2 ppb Methylphosphonsäure-Dimethylester

-Totzeit: < 0,5 s

-95 %ige Ansprechzeit:<5 s

-95 %ige Rückstellzeit:<5 Minuten

-Querempfindlichkeit: gegen $SO_2$ ca. 500, gegen Kohlenwasserstoffe ca. $10^5$.


Aufstellung der verwendeten Bezugszeichen

1 - Aerosolfilter

2 - Ofen

3 - Ionisationskammer

4 - Widerstand

5 - Impedanzwandler

6 - Anzeigegerät

7 - Strömungsmesser

8 - Ventil

9 - Pumpe

10 - Kapillare

11 - Absorptionsfilter

12 - Bestrahlungsgefäß


**Ansprüche**

1. Verfahren zum Nachweis phosphorhaltiger Verbindungen in Luft durch deren Umwandlung in Aerosole und die ionisationsgasanalytische Bestimmung der Aerosolkonzentration, dadurch gekennzeichnet, daß auf die phosphorhaltigen Verbindungen, in Gegenwart von Sauerstoff und/oder Wasserdampf thermische, Strahlungs-oder chemische Energie übertragen wird.

2. Verfahren nach Punkt 1, dadurch gekennzeichnet, daß bei Zuführung thermischer Energie der Temperaturgradient in der anschließenden Kondensationszone -(200 ... 300) K.cm$^{-1}$.s$^{-1}$ beträgt.

3. Verfahren nach Punkt 1 und 2, dadurch gekennzeichnet, daß die thermische Energie so gewählt wird, daß der Phosphor nur aus bestimmten Bindungen in den nachzuweisenden Verbindungen herausgelöst wird.

4. Verfahren nach Punkt 1 und 2, dadurch gekennzeichnet, daß die thermische Energie so hoch gewählt wird, daß alle Bindungen des Phosphors in den nachzuweisenden Verbindungen gelöst werden.

5. Verfahren nach Punkt 1, dadurch gekennzeichnet, daß ein Teil des Meßgases von den phosphorhaltigen Verbindungen gereinigt und durch eine stille Wechselspannungsentladung geleitet oder mit UV-C-Strahlung behandelt und dann wieder mit dem Meßgas vermischt wird.

6. Verfahren nach Punkt 1, dadurch gekennzeichnet, daß natürlich vorhandene Aerosole vor der Behandlung des Meßgases entfernt werden.

7. Verfahren nach Punkt 1, dadurch gekennzeichnet, daß die Konzentration der entstehenden Aerosole ionisationsgasanalytisch gemessen wird.

8. Verfahren nach Punkt 1, dadurch gekennzeichnet, daß anstelle von Luft als Trägergas andere Gase eingesetzt werden und erforderlichenfalls Sauerstoff und/oder Wasserdampf zugesetzt wird.

Fig.1

Fig.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | PLASMA CHEMISTRY AND PLASMA PROCESSING, Band 4, Nr. 1, März 1984, Seiten 15-20, Bristol, GB; E.J. CLOTHIAUX et al.: "Decomposition of an organophosphorus material in a silent electrical discharge" * ganzes Dokument * | 1,5,7, 8 | G 01 N 27/66 |
| Y | DD-A- 214 213 (AKADEMIE DER WISSENSCHAFTEN DER DDR) * Zusammenfassung; Seite 2, Zeile 7 - Seite 3, Zeile 18; Seite 4, Zeile 18 - Seite 5, Zeile 6; Ansprüche * | 1,5,7, 8 | |
| A | US-A-3 547 588 (J. MIYAMOTO et al.) * ganzes Dokument * | 1,2 | |
| | | | RECHERCHIERTE SACHGEBIETE (Int. Cl.4) |
| A | GB-A-1 604 926 (PYE(ELECTRONIC PRODUCTS) LTD.) * Seite 1, Zeilen 5-9; Zeilen 22-37; Seite 2, Zeilen 29-35; Ansprüche * | 1,6-8 | G 01 N 27/00 |
| A | GB-A-1 604 925 (PYE(ELECTRONIC PRODUCTS)LTD.) * Seite 1, Zeilen 9-17; Zeilen 43-70; Seite 2, Zeilen 35-90; Ansprüche * | 1,6-8 | |

--- -/-

Der vorliegende Recherchenbericht wurde für alle Patentansprüche erstellt.

| Recherchenort BERLIN | Abschlußdatum der Recherche 27-03-1987 | Prüfer VINSOME R M |
|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| A | EP-A-0 135 135 (HONEYWELL INC.) <br> * ganzes Dokument * <br><br> --- | 1,8 | |
| A | DE-C-3 409 932 (NIESSNER et al.) <br> * ganzes Dokument * <br><br> --- | 1 | |
| A | DE-A-1 926 937 (VEB JUNKALOR DESSAU) <br><br> ----- | | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| BERLIN | 27-03-1987 | VINSOME R M |